# EUROPEAN PATENT SPECIFICATION

(11) **EP 0 691 814 B1**
(45) Date of publication and mention of the grant of the patent: **26.06.2002**
(21) Application number: 94912337.6
(22) Date of filing: 29.03.1994
(51) Int. Cl.: A01N 59/00, A61K 33/10, A61K 31/54, A61K 31/19

(54) **METHOD AND COMPOSITION FOR TREATMENT OF OSTEOPOROSIS**
VERFAHREN UND ZUSAMMENSETZUNGEN ZUR BEHANDLUNG DER OSTEOPOROSE
PROCEDE ET COMPOSITION S'APPLIQUANT AU TRAITEMENT DE L'OSTEOPOROSE

(30) Priority: 02.04.1993 US 42296
(43) Date of publication of application: 17.01.1996
(73) Proprietor: THE REGENTS OF THE UNIVERSITY OF CALIFORNIA, Oakland, California 94612-3550 (US)
(72) Inventor: MORRIS, R. Curtis, Jr., San Francisco, CA 94123 (US); SEBASTIAN, Anthony, San Francisco, CA 94131 (US)
(74) Representative: Brown, John David
(86) International application number: US9403403
(87) International publication number: WO9422312

(56) References cited:
- DE-A- 3 241 765
- US-A- 4 965 282
- US-A- 4 966 776

## Description

### FIELD OF THE INVENTION

This invention concerns a composition useful for treating osteoporosis with a combination of an alkalinizing potassium salt and a thiazide diuretic which is effective in such treatment, which drug combination reduces the health risks or side effects associated with thiazide treatment.

### BACKGROUND OF THE INVENTION

Osteoporosis is a metabolic bone disease
characterized pathologically by an absolute decrease in the amount of bone, and clinically by increased susceptibility to fractures. Riggs et al., N. Engl. J. Med. (1986), 314:1676; Rusbach et al., in: Textbook of Endocrinology, Ed(s) Williams, (1981), p. 922; Riggs, in: Cecil Textbook of Medicine, Ed(s) Wyngaarden et al., (1985), p. 1456; Riggs et al., Am. J. Med., (1983), 75:899.

There are several biochemical markers which taken together, can be used to either diagnose a patient as osteoporotic, or to study the efficacy of treatments for osteoporosis. For example, urinary hydroxyproline excretion rate is widely used as a marker for bone resorption. Klein et al., Metabolism 2, Vol. 13, No. 3, March 1964, 272-285; Charles et al., J. Clin. Invest., Vol. 76, December 1985 2254-2258; and Deacon et al., Clin. Chim. Acta., 1987, 297-306. Pyridinoline and deoxy-pyridinoline, two types of collagen crosslinks present in bone, which can be detected in urine, are also markers for bone resorption. Robins, et al., European Journal of Clinical Investigation (1991), 21:310-315.

Serum concentrations of osteocalcin serve as a biochemical marker of the rate of bone formation. Osteocalcin is an integral protein of the organic matrix of bone synthesized by bone-forming cells (osteoblasts) during the process of bone formation. A small fraction of the newly synthesized osteocalcin escapes into the circulatory system, thus providing a blood marker of the rate of bone formation. The osteocalcin concentration increases when the bone formation rate increases, and decreases when the bone formation rate decreases. Brown, et al., The Lancet, May 19, 1984, p. 1091, "Serum Bone GLA-Protein: A Specific Marker For Bone Formation in Postmenopausal Osteoporosis". Another reflection of bone resorption/bone formation are changes in calcium and phosphorus balances (positive or negative) which are determined by measuring the difference between the total excretion (feces and urine) and the dietary intake of calcium or phosphorus ion. (These balances are positive when the total excretion is less than the dietary intake.)

Thiazide diuretics are widely used for the treatment of hypertension. In recent years a number of studies have suggested that they may also have a potential role in the prevention of bone loss and osteoporotic fracture, leading to several recent proposals for randomized, controlled clinical trials thereof (Lacroix, Comprehensive Therapy (1991), 17(8): 30-39; Editorial, "Thiazide Diuretics and Osteoporosis", BJCP, Autumn 1991, 45(3); Ray, W.A., Editorial, "Thiazide Diuretics and Osteoporosis: Time for a Clinical Trial?", 1 July 1991, Annals of Internal Medicine 115(1): 64-65).

The proposals that the thiazide diuretics may be effective in the treatment of osteoporosis are based on the recognition that they reduce urinary calcium excretion (Adland-Davenport et al., Am. J. Obstet. Gynecol., (July 15, 1985), 152(6) Part 1: 630-634; Wasnich et al., Obstetrics and Gynecology, (April 1986), 67(4): 457-462; Ray et al., The Lancet, (April 1, 1989): 687-690; Steiniche et al., APMIS, (1989), 97:302-308; and Lacroix et al., New Enq. J. Med., (Feb. 1, 1990): 286-290), improve calcium balance (Wasnich et al., New Eng, J. Med., (Aug. 11, 1983): 344-347; Hunt et al., Am. J. Clin, Nutr., (1989), 50: 517-523; Steiniche et al., 1989; and La Croix et al., 1990) and decrease bone loss (Wasnich et al., Br. Med. J., (1990), 301: 1303-1305), coupled with the recent reported studies associating thiazide use with a decreased risk of hip fracture (Ray et al., 1989; LaCroix et al., 1990; and Felson et al., JAMA, (1991), 265: 370-373).

The thiazide diuretics have, however, been associated with a recognized set of side effects, particularly when administered at higher doses. Administration of the thiazides commonly causes hypokalemia (Bloomfield et al., 1986, J. Clin. Hypertens, 4:331-338; Solomon et al., J. Cardiovasc, Pharmacol. (1991) 17:854-859). They cause postural hypotension, resulting in increased frequency of fainting, dizziness and loss of consciousness in women (La Croix, 1991; Hale et al., J. Am. Geriatric Soc, (1984) 32:5-10). In men, impotence commonly occurs (Papadopoulos, Arch, Intern. Med., Vol. 140, p. 1341 (1980); and Report of Medical Research Council Working Party on Mild to Moderate Hypertension, The Lancet, Sept. 12, 1981, pp. 539-543). In addition, they may adversely affect electrolytes, carbohydrate metabolism, lipids and kidney function (Fried et al., in Diuretics Physiology Pharmacology and Clinical Use, 1986, Chapter 4, pp. 66-82).

It is therefore desirable to take advantage of the hypocalciuric properties of the thiazide diuretics in the treatment of osteoporotic disease, while avoiding the multiple side effects thereof.

U.S. Patent No. 5,171,583, granted on December 15, 1992, discloses a method for ameliorating or preventing osteoporosis in humans afflicted with or predisposed to osteoporosis, comprising administering a composition containing a therapeutically or prophylactically-erfective amount of a composition of a pharmaceutically-acceptable alkalinizing potassium salt. An effective dose of the alkalinizing potassium salt of 40-400 mmoles/70kg patient weight/day and preferably 40-250 mmoles/70kg/day is disclosed therein.

In accordance with the present invention, when the thiazide diuretics are used in combination with the foregoing alkalinizing potassium salts, a use and composition for treating osteoporosis is provided which reduces if not eliminates hypokalemia and the risks and side effects associated with the use of the thiazides.

### SUMMARY OF THE INVENTION

According to the present invention, there is provided a combination of two drugs for treating osteoporotic disease in a human being subject thereto, which comprises a synergistically effective dosage of the following active ingredients:
(a) a pharmacologically-acceptable alkalinizing potassium salt capable of reducing the acidity of tissue fluids or urine, said alkalinizing potassium salt being selected from the group consisting of potassium bicarbonate and potassiumn salts of carboxylic acids which alkalinize in vivo; and
(b) a thiazide diuretic.

Also provided by the present invention is the use of a combination of two drugs comprising a synergistically effective dosage of the following active ingredients:
(a) a pharmacologically-acceptable alkalinizing potassium salt capable of reducing the acidity of tissue fluids or urine, said alkalinizing potassium salt being selected from the group consisting of potassium bicarbonate and potassium salts of carboxylic acids which alkalinize in vivo; and
(b) a thiazide diuretic,
   for the manufacture of a medicament for use in treating osteoporotic disease in a human being subject thereto.

Also disclosed is a combination useful for ameliorating or preventing osteoporosis in humans afflicted with or predisposed to osteoporosis, which comprises an effective dosage of a combination drug comprising the following active ingredients:
(a) a pharmacologically-acceptable alkalinizing potassium salt which produces hydroxyl ions and is thereby capable of reducing the acidity (by increasing the alkalinity) of tissue fluids or urine and which is selected from the group consisting of potassium bicarbonate and potassium salts of carboxylic acids which are transformed (combusted) to bicarbonate and thus alkalinize in vivo; and (b) a thiazide diuretic.

The alkalinizing potassium salt may be administered in an amount of from 30 to 180 milliequivalents/70 kg patient weight/day, for example, in an amount of 60 to 120 milliequivalents per day. The thiazide diuretic may be administered in amounts ranging from 10% to 90% of the minimum usual daily oral diuretic dose in humans.

In a preferred embodiment of the invention, the alkalinizing potassium salt and thiazide diuretic are for administration in a plurality of unit dosage forms equivalent to a daily dosage of from 30 to 180 milliequivalents of the alkalinizing potassium salt and an amount of the thiazide diuretic equivalent to from 3 to 20 mgs. of hydrochlorothiazide. Preferably, the alkalinizing potassium salt is for administration in a daily dosage equivalent of from 60 to 120 milliequivalents.

Where the thiazide is incorporated in amounts ranging from 10% to 90% of its minimum usual daily oral diuretic dose in humans, the alkalinizing potassium salt may be present in the composition in an amount such that the composition
(a) reduces the urinary hydroxyproline excretion rate of the human being treated more than 10% relative to the urinary hydroxyproline excretion rate of the human being observed when the same amount of the thiazide diuretic is independently administered;
(b) reduces the urinary collagen cross-link excretion rate of the human being treated by more than 10% relative to the urinary collagen cross-link excretion rate of the human being when the same amount of the thiazide diuretic is independently administered; and
(c) increases the serum osteocalcin concentration of the human being treated by more than 10% relative to the serum osteocalcin concentration of the human being when the same amount of the thiazide diuretic is independently administered.

In a preferred embodiment of the invention, the alkalinizing potassium salt is present in the composition in an amount such that the composition
(a) reduces the urinary hydroxyproline excretion rate of the human being treated more than 10% relative to the urinary hydroxyproline excretion rate of the human being observed when the same amount of the thiazide diuretic is independently administered;
(b) reduces the urinaly collagen cross-link excretion rate of the human being treated by more than 10% relative to the urinary collagen cross-link excretion rate of the human being when the same amount of the thiazide diuretic is independently administered; and
(c) increases the calcium and phosphorus balances of the human being treated by more than 10% relative to the balances when the same amount of the thiazide diuretic is independently administered.

By combining the pharmacologically-acceptable alkalinizing potassium salt and the thiazide diuretic, unexpectedly superior results are obtained in the treatment of osteoporosis as compared with the results obtained by treatment with either such active ingredient alone. Otherwise stated, it is possible in some instances to decrease the amount of the thiazide diuretic ingredient by as much as 90% without materially diminishing the hypocalciuric effect obtained by it when administered as the sole active ingredient. The combination drug of the present invention thus provides the significant benefit of reducing, if not eliminating, the health risks and side effects which may be associated with administration of the thiazide diuretic.

### DETAILED DESCRIPTION OF THE INVENTION

The two active ingredients of the combination drug invention, i.e., (a) the pharmacologically acceptable alkalinizing potassium salt and (b) the thiazide diuretic, may be administered as separate dosage forms in conjunction with one another. Alternatively, and preferably, as described more fully below, the alkalinizing potassium salt may be combined with the thiazide in a unitary dosage form which can be administered to subjects without the need for independent administration of these active ingredients.

As used herein, the terms "treatment" or "treating" cover any treatment of osteoporotic disease, and include: (1) preventing osteoporosis from occurring in a subject who does not have osteoporosis or who has not yet been diagnosed as having it; (2) inhibiting or arresting the development of the disease; or (3) regressing or reversing the osteoporotic state.

As further used herein, the combination drug of the invention is utilized in an "effective dosage" when it causes the following effects in the patient:
(a) it reduces the urinary hydroxyproline excretion rate;
(b) it reduces the urinary collagen crosslink excretion rate; and
(c) it increases calcium and phosphorus balances, i.e., makes them less negative or more positive.

As also used herein, the term "collagen crosslinks" means pyridinoline and deoxy-pyridinoline crosslinks.

Finally, as used herein the term "calcium balance" means the difference between the total excretion (feces and urine) of calcium and the dietary intake of calcium ion. Similarly, the term "phosphorus balance" means the difference between the total excretion (feces and urine) of phosphorus and the dietary intake of phosphorus ion.

The alkalinizing potassium salts which may be employed in the composition of the present invention are those which, when present in the body fluids, produce hydroxyl ions and are thereby capable of reducing the acidity (increasing the alkalinity) of tissue fluids or urine. A number of pharmaceutically-acceptable alkalinizing potassium salts are known, several of which are set forth in Berg et al., J. Pharmaceut. Sci. (1977) 66:1. Given the disclosure herein, it will be well within the ability of one skilled in the art to select and screen pharmaceutically-acceptable alkalinizing salts for the ability to treat osteoporosis using well known methods and techniques. Desirably, a salt will be selected which is therapeutically effective in amounts readily achievable in humans while being relatively well tolerated. Different salts may be chosen depending on particular routes of administration and preferred modes of formulation.

The alkalinizing potassium salts which may be thus administered are preferably selected from the group consisting of potassium bicarbonate (KHCO₃) and pharmacologically acceptable, non-toxic potassium salts of carboxylic acids such as potassium gluconate (C₆H₁₁KO₇) and potassium citrate (C₆H₅K₃O₇). The use of potassium bicarbonate is particularly preferred. The preparation, isolation and purification of these salts are well known to those skilled in the art, as they are commonly employed in a therapeutic setting for a variety of uses other than described herein. Specific procedures for the preparation of such salts are described in general terms in Remington's Pharmaceutical Sciences, Mack Publishing Company, Easton, Pennsylvania, 16th Ed., 1982.

The thiazide diuretics useful in the composition of the present invention comprise any of those conventionally utilized in the treatment of hypertension. Such agents are identified in Goodman and Gilman, The Pharmacological Basis of Therapeutics, Eighth Edition, 1990, pp. 718-721 and 785-788; and in Drug Evaluations Subscription, AMA Div. of Drugs and Toxicology, 1992, Vol. II - Renal-Urologic Drugs, in Table 5. As used herein, the "thiazide diuretics" include the sulfonamide diuretics, e.g., chlorthalidone, whose pharmacological action is indistinguishable from that of the thiazides (See Goodman and Gilman, supra, at page 718). Preferred thiazide diuretics useful herein include chlorothiazide, hydrochlorothiazide, and chlorthalidone.

The thiazide diuretics are believed to be especially effective in the treatment of osteoporotic disease, in combination with the alkalinizing potassium salts, since they are weak carbonic anhydrase inhibitors which impair hydrogen ion secretion and thus decrease osteoclast resorption of bone. Although carbonic anhydrase inhibitors also produce acidosis, it is believed that the alkalinizing potassium salt prevents acidotic conditions and thus acts synergistically with the preferred thiazide diuretics, minimizing the amount of the latter required to produce a hypocalciuric effect. It should, however, be understood that the present invention is not limited by the foregoing hypothesized mechanism of co-action between the active ingredients of the preferred composition.

Administration of the pharmacologically acceptable alkalinizing potassium salt or the thiazide diuretic ingredients of the combination drug of the present invention may be in pharmaceutical compositions described hereinafter and can be via any of the accepted modes of administration for agents which are known to be useful in the treatment of osteoporosis. Each such ingredient may be administered orally, parenterally, or otherwise. Different active alkalinizing potassium salts or thiazide diuretics may be admixed and simultaneously administered, or benefit may be gained in some instances by their separate, sequential administration.

Depending on the intended mode, the alkalinizing potassium salt ingredient may be in the form of solid, semisolid or liquid dosage forms, such as, for example, tablets, capsules, pills, powders, granules, crystals, liquids, suspensions, or the like, preferably in unit-dosage forms suitable for administration of relatively precise dosages. Similarly, the thiazide diuretic ingredient may be in the form of a solid tablet, capsule or pill, preferably in unit-dosage forms suitable for administration of relatively precise dosages.

Preferably, the alkalinizing salt and the thiazide active ingredients are combined in a solid unitary dosage form in a tablet, capsule or pill, thus obviating the need for separate administration of these ingredients. The solid combined dosage form may include conventional pharmaceutical carriers or excipients, and, in addition, may include other pharmaceutical agents. Thus, the unit dosage form may be compounded with conventional nontoxic solid carriers such as, for example, pharmaceutical grades of mannitol, lactose, starch, magnesium stearate, talcum, cellulose, glucose, sucrose, magnesium carbonate, and the like. Such compositions may contain about 50-90% of the active ingredients of the present invention, preferably about 70-90%.

Alternatively, the alkalinizing potassium salt ingredient may be administered as a separate dosage form, in conjunction with the administration of the thiazide diuretic. The two drugs may thus be administered on the same schedule or on different schedules in accordance with the normal modes of administration thereof. When the alkalinizing potassium salt ingredient is administered as a separate dosage form, it may be in the form of tablets, pills, capsules, powders, granules, crystals, sustained-release formulations, and the like, with any of the previously listed excipients, or may be administered in a liquid pharmaceutically-administrable composition. Such liquid compositions can be prepared, for example, by dissolving the salt, such as potassium bicarbonate, and optional pharmaceutical adjuvants in a carrier, such as, for example, water, aqueous dextrose, glycerol, and the like, to thereby form a solution or suspension. If desired, the separate alkalinizing salt dosage form may also contain minor amounts of nontoxic auxiliary substances such as pH buffering agents and the like, for example, sorbitan monolaurate, triethanolamine, sodium acetate, triethanolamine oleate, etc. Actual methods of preparing such dosage forms are known, or will be apparent, to those skilled in this art; see, for example, the aforesaid Remington's Pharmaceutical Sciences, Mack Publishing Company, Easton, Pennsylvania, 16th Ed., 1982. An active potassium salt ingredient of the combination drug, such as potassium bicarbonate, for example, may be provided as a dietary supplement supplied as pills, as granules or powder applied directly to foodstuffs, or dissolved in drinking water, as convenient means of administration.

Preferably, the thiazide ingredient of the combination drug is administered in a daily dosage equivalent to 3 to 20 mg of hydrochlorothiazide and the alkalinizing potassium salt is administered in a daily dosage of 30-180, preferably 60 to 120 milliequivalents. At these levels, the following relative effects are observable in comparison to the effects when the same amount of the thiazide diuretic is separately administered:
(a) a reduction in the urinary hydroxyproline excretion rate (measured as described in the Klein, Charles et al. and Deacon et al. publications described above) by more than 10%;
(b) a reduction in the urinary collagen crosslink excretion rate (measured as described in the Robins et al. publication described above) by more than 10%; and
(c) an increase in calcium and phosphorus balances (measured as described in conventional manner), for example, by as much as 10%, or more.

As can be seen from the foregoing, the combination drug of the present invention exhibits a synergistic effect in treating osteoporosis, i.e., the combination of the two drugs is substantially more effective than the same amount of the thiazide when independently administered. Most preferably, the thiazide diuretic ingredient of the combination drug may be administered in an amount equivalent to the daily oral administration of 3 to 20 mg of hydrochlorothiazide, appreciably lower than the usual daily oral diuretic dose in humans. The combination of the present invention incorporating these lower dosages of the thiazide is not only effective in treating osteoporosis, but also significantly reduces the health risks and side effects associated with thiazide diuretics at higher doses. It is generally convenient to supply the foregoing daily dosages in multiple (e.g., 3 to 5) tablets incorporating the active ingredients in suitable excipients and coated with a suitable sustained release coating.

The amount of the thiazide diuretic administered in accordance with the present invention will, of course, be dependent on the potency of the particular thiazide used and the mode of administration. The relative and equivalent potencies of various thiazide diuretics are well known to those skilled in the art. (The equivalent daily diuretic doses of hydrochlorothiazide and other thiazide diuretics are disclosed in Fried et al., 1986, supra, at pp. 68-70 and in Drug Evaluations Subscription, supra). For example, 500 mg of chlorothiazide are equivalent to 75 mg of hydrochlorothiazide in terms of hypocalciuric potency and ability to treat osteoporosis. Given the disclosure herein, it will be well within the ability of one skilled in the art to select a thiazide diuretic and a dose level equivalent to the dosages of the particular thiazides described herein.

It will also be appreciated by those having skill in the art that in addition to administering the combination drug described herein, it may be desirable to supplement the patient's calcium intake, if necessary, to maintain it at about 1500 mg of calcium per day.

The following examples illustrate some particularly preferred, non-limiting embodiments of the present invention.

### EXAMPLE I

A combination drug tablet is prepared containing the following active ingredients: 3 mg of hydrochlorothiazide and 1.5 grams of potassium bicarbonate. Four such tablets are administered daily, to provide a daily dose of 12 mg of the hydrochlorothiazide and 6.0 grams (60 milliequivalents) of the potassium bicarbonate.

### EXAMPLE II

A combination drug tablet is prepared containing the same ingredients as Example I, except that the tablet contains only 0.75 mg of hydrochlorothiazide. Again, four such tablets are administered daily for effective treatment of osteoporosis.

From the foregoing, it will be appreciated that the present invention relates to a novel use and composition which effectively treats/prevents osteoporosis in human subjects, with lower health risks and incidence of side effects than would be associated with thiazide diuretics.

## Claims

1. A combination of two drugs for treating osteoporotic disease in a human being subject thereto, which comprises a synergistically effective dosage of the following active ingredients:
(a) a pharmacologically-acceptable alkalinizing potassium salt capable of reducing the acidity of tissue fluids or urine, said alkalinizing potassium salt being selected from the group consisting of potassium bicarbonate and potassiumn salts of carboxylic acids which alkalinize in vivo; and
(b) a thiazide diuretic

2. The combination of Claim 1, wherein the thiazide diuretic is selected from the group consisting of chlorothiazide, hydrochlorothiazide and chlorothalidone.

3. The combination of Claim 1, in which the thiazide is incorporated in amounts ranging from 10% to 90% of its minimum usual daily oral diuretic dose in humans.

4. The combination of Claim 3, wherein the alkalinizing potassium salt is present in the composition in an amount such that the composition
(a) reduces the urinary hydroxyproline excretion rate of the human being treated more than 10% relative to the urinary hydroxyproline excretion rate of the human being observed when the same amount of the thiazide diuretic is independently administered;
(b) reduces the urinary collagen crosslink excretion rate of the human being treated by more than 10% relative to the urinary collagen crosslink excretion rate of the human being when the same amount of the thiazide diuretic is independently administered; and
(c) increases the calcium and phosphorus balances of the human being treated by more than 10% relative to the balances when the same amount of the thiazide diuretic is independently administered.

5. A combination according to any one of Claims 1 to 4, which further comprises a pharmaceutically-acceptable carrier.

6. A combination according to any one of Claims 1 to 5, wherein the alkalinizing potassium salt is potassium bicarbonate.

7. The combination of Claim 1, wherein the alkalinizing potassium salt and the thiazide diuretic are for administration in a plurality of unit dosage forms equivalent to a daily dosage of from 30 to 180 milliequivalents of the alkalinizing potassium salt and an amount of the thiazide diuretic equivalent to from 3 to 20 mgs. of hydrochlorothiazide.

8. The combination of Claim 7, wherein the alkalinizing potassium salt is for administration in a daily dosage equivalent of from 60 to 120 milliequivalents.

9. The combination of Claim 7 or 8, wherein the alkalinizing potassium salt and the thiazide diuretic are for administration in separate unit dosage forms.

10. The combination of Claim 7 or 8, wherein the alkalinizing potassium salt and the thiazide diuretic are for administration in admixture in a unitary dosage form.

11. A combination according to any one of Claims 7 to 10, wherein the unitary dosage form further comprises a pharmaceutically-acceptable carrier.

12. A combination according to any one of Claims 7 to 11, wherein the alkalinizing potassium salt is potassium bicarbonate.

13. Use of a combination of two drugs comprising a synergistically effective dosage of the following active ingredients:
(a) a pharmacologically-acceptable alkalinizing potassium salt capable of reducing the acidity of tissue fluids or urine, said alkalinizing potassium salt being selected from the group consisting of potassium bicarbonate and potassiumn salts of carboxylic acids which alkalinize in vivo; and
(b) a thiazide diuretic,
for the manufacture of a medicament for use in treating osteoporotic disease in a human being subject thereto.

## Patentansprüche

1. Kombination von zwei Wirkstoffen zur Behandlung von Osteoporose-Erkrankung bei einem Menschen, der an dieser leidet, welche eine synergistisch wirksame Dosierung der folgenden aktiven Inhaltsstoffe umfaßt:
(a) ein pharmakologisch annehmbares alkalisierendes Kaliumsalz, das in der Lage ist, die Azidität von Gewebeflüssigkeiten oder Urin zu verringern, wobei besagtes alkalisierendes Kaliumsalz ausgewählt ist aus der Gruppe, bestehend aus Kaliumbicarbonat und Kaliumsalzen von Carbonsäuren, die in vivo alkalisieren; und
(b) ein Thiazid-Diuretikum.

2. Kombination nach Anspruch 1, **dadurch gekennzeichnet, daß** das Thiazid-Diuretikum ausgewählt ist aus der Gruppe, die aus Chlorthiazid, Hydrochlorthiazid und Chlorthalidon besteht.

3. Kombination nach Anspruch 1, **dadurch gekennzeichnet, daß** in ihr das Thiazid in Mengen einbezogen ist, die von 10 % bis 90 % seiner minimalen üblichen täglichen oralen diuretischen Dosis bei Menschen reichen.

4. Kombination nach Anspruch 3, **dadurch gekennzeichnet, daß** das alkalisielende Kaliumsalz in der Zusammensetzung in einer solchen Menge vorhanden ist, daß die Zusammensetzung
(a) die Hydroxyprolin-Urinausscheidungsrate des behandelten Menschen mehr als 10 % relativ zu der Hydroxyprolin-Urinausscheidungsrate des Menschen verringert, die beobachtet wird, wenn dieselbe Menge des Thiazid-Diuretikurns unabhängig verabreicht wird;
(b) die Collagenvernetzungs-Urinausscheidungsrate des behandelten Menschen um mehr als 10 % relativ zu der Collagenvernetzungs-Urinausscheidungsrate des Menschen verringert, die vorliegt, wenn dieselbe Menge des Thiazid-Diuretikums unabhängig verabreicht wird; und
(c) die Calcium- und Phosphor-Gleichgewichte des behandelten Menschen um mehr als 10 % relativ zu den Gleichgewichten erhöht, wenn dieselbe Menge des Thiazid-Diuretikums unabhängig verabreicht wird.

5. Kombination nach einem der Ansprüche 1 bis 4, die weiter einen pharmazeutisch annehmbaren Trägerstoff umfaßt.

6. Kombination nach einem der Ansprüche 1 bis 5, **dadurch gekennzeichnet, daß** das alkalisierende Kaliumsalz Kaliumbicarbonat ist.

7. Kombination nach Anspruch 1, **dadurch gekennzeichnet, daß** das alkalisierende Kaliumsalz und das Thiazid-Diuretikum zur Verabreichung in einer Mehrzahl von Dosiseinheitsformen gedacht sind, die äquivalent sind zu einer täglichen Dosis von 30 bis 180 Milliäquivalenten des alkalisierenden Kaliumsalzes und einer Menge des Thiazid-Diuretikums, die äquivalent ist zu von 3 bis 20 mg Hydrochlorthiazid.

8. Kombination nach Anspruch 7, **dadurch gekennzeichnet, daß** das alkalisierende Kaliumsalz zur Verabreichung in einer täglichen Dosis gedacht ist, die äquivalent ist zu von 60 bis 120 Milliäquivalenten.

9. Kombination nach Anspruch 7 oder 8, **dadurch gekennzeichnet, daß** das alkalisierende Kaliumsalz und das Thiazid-Diuretikum zur Verabreichung in separaten Dosiseinheitsformen gedacht sind.

10. Kombination nach Anspruch 7 oder 8, **dadurch gekennzeichnet, daß** das alkalisierende Kaliumsalz und das Thiazid-Diuretikum zur Verabreichung in Vermischung in einer einheitlichen Dosisform gedacht sind.

11. Kombination nach einem der Ansprüche 7 bis 10, **dadurch gekennzeichnet, daß** die einheitliche Dosisform außerdem einen pharmazeutisch annehmbaren Trägerstoff umfaßt.

12. Kombination nach einem der Ansprüche 7 bis 11, **dadurch gekennzeichnet, daß** das alkalisierende Kaliumsalz Kaliumbicarbonat ist.

13. Verwendung einer Kombination von zwei Wirkstoffen, die eine synergistisch wirksame Dosierung der folgenden aktiven Inhaltsstoffe umfaßt:
(a) ein pharmakologisch annehmbares alkalisierendes Kaliumsalz, das in der Lage ist, die Azidität von Gewebeflüssigkeiten oder Urin zu verringern, wobei besagtes alkalisierendes Kaliumsalz ausgewählt ist aus der Gruppe, bestehend aus Kaliumbicarbonat und Kaliumsalzen von Carbonsäuren, die in vivo alkalisieren; und
(b) ein Thiazid-Diuretikum,
zur Herstellung eines Arzneimittels zur Velwentung bei der Behandlung von Osteoporose-Erkrankung bei einem Menschen, der an dieser leidet.

## Revendications

1. Association de deux médicaments destinée à traiter une maladie ostéoporotique chez un être humain qui y est sujet; qui comprend une dose efficace en synergisme des ingrédients actifs suivants :
(a) un sel de potassium alcalinisant acceptable d'un point de vue pharmacologique capable de réduire l'acidité des liquides tissulaires ou de l'urine, ledit sel de potassium alcalinisant étant choisi dans le groupe consistant en bicarbonate de potassium et les sels de potassium d'acides carboxyliques qui ont un effet alcalinisant *in vivo ;* et
(b) un diurétique thiazidique.

2. Association selon la revendication 1, dans laquelle le diurétique thiazidique est choisi dans le groupe consistant en chlorothiazide, hydrochlorothiazide et chlortalidone.

3. Association selon la revendication 1, dans laquelle le diurétique thiazidique est incorporé dans des quantités comprises dans l'intervalle de 10% à 90% de sa dose journalière orale diurétique minimale habituelle chez l'être humain.

4. Association selon la revendication 3, dans laquelle le sel de potassium alcalinisant est présent dans la composition dans une quantité telle que la composition :
(a) réduit le taux d'excrétion urinaire d'hydroxyproline chez l'être humain traité de plus de 10% par rapport au taux d'excrétion urinaire d'hydroxyproline que l'on observe chez ledit être humain lorsque la même quantité du diurétique thiazidique est administrée indépendamment ;
(b) réduit le taux d'excrétion urinaire de collagène réticulé chez l'être humain de plus de 10% par rapport au taux d'excrétion urinaire de collagène réticulé que l'on observe chez ledit être humain lorsque la même quantité du diurétique thiazidique est administrée indépendamment ; et
(c) augmente les bilans calcique et phosphorique chez l'être humain traité de plus de 10% par rapport aux bilans que l'on observe chez ledit être humain lorsque la même quantité du diurétique thiazidique est administrée indépendamment.

5. Association selon l'une quelconque des revendications 1 à 4, qui comprend en outre un véhicule acceptable d'un point de vue pharmaceutique.

6. Association selon l'une quelconque des revendications 1 à 5, dans laquelle le sel de potassium alcalinisant est le bicarbonate de potassium.

7. Association selon la revendication 1, dans laquelle le sel de potassium alcalinisant et le diurétique thiazidique sont destinés à être administrés dans une pluralité de formes pharmaceutiques unitaires équivalentes à une dose journalière allant de 30 à 180 milli-équivalents du sel de potassium alcalinisant et une quantité du diurétique thiazidique équivalente à de 3 à 20 mg d'hydrochlorothiazide.

8. Association selon la revendication 7, dans laquelle le sel de potassium alcalinisant est destiné à être administré à une dose journalière équivalant à de 60 à 120 milli-équivalents.

9. Association selon la revendication 7 ou 8, dans laquelle le sel de potassium alcalinisant et le diurétique thiazidique sont destinés à être administrés dans des formes pharmaceutiques distinctes.

10. Association selon la revendication 7 ou 8, dans laquelle le sel de potassium alcalinisant et le diurétique thiazidique sont destinés à être administrés en mélange dans une forme pharmaceutique unitaire.

11. Association selon l'une quelconque des revendications 7 à 10, dans laquelle la forme pharmaceutique unitaire comprend en outre un véhicule acceptable d'un point de vue pharmaceutique.

12. Association selon l'une quelconque des revendications 7 à 11, dans laquelle le sel de potassium alcalinisant est le bicarbonate de potassium.

13. Utilisation d'une association de deux médicaments comprenant une dose efficace en synergisme des ingrédients actifs suivants :
(a) un sel de potassium alcalinisant acceptable d'un point de vue pharmacologique capable de réduire l'acidité des liquides tissulaires ou de l'urine, ledit sel de potassium alcalinisant étant choisi dans le groupe consistant en bicarbonate de potassium et les sels de potassium d'acides carboxyliques qui ont un effet alcalinisant *in vivo* ; et
(b) un diurétique thiazidique,
pour la fabrication d'un médicament destiné à être utilisé dans le traitement d'une maladie ostéoporotique chez un être humain qui y est sujet.
